(19) Europäisches Patentamt
European Patent Office
Office européen des brevets

(11) **EP 4 082 518 A1**

(12) **EUROPEAN PATENT APPLICATION**

(43) Date of publication:
**02.11.2022 Bulletin 2022/44**

(21) Application number: **21382370.1**

(22) Date of filing: **28.04.2021**

(51) International Patent Classification (IPC):
*A61K 8/36* (2006.01)        *A61Q 7/00* (2006.01)

(52) Cooperative Patent Classification (CPC):
**A61Q 7/00; A61K 8/361;** A61K 2800/5922

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **CNCE INNOVACION, S.L.**
**08015 Barcelona (ES)**

(72) Inventor: **REY, Francisco Javier Morán**
**28033 Madrid (ES)**

(74) Representative: **Isern Patentes y Marcas S.L.**
**Avda. Diagonal, 463 Bis, 2°**
**08036 Barcelona (ES)**

(54) **FATTY ACID COMPOSITIONS FOR THE TREATMENT AND PREVENTION OF HAIR LOSS AND ALOPECIA**

(57)    The present invention related to fatty acid mixture with improved efficacy and inhibited gene expression of SRD5A1, SRD5A2 and SRD5A3 as well as compositions thereof suitable for the treatment and/or prevention of hair loss and alopecia, notably with lower dosage and low toxicity. Furthermore, the present invention also relates to the process to obtain said mixtures and composition and to the use of said compositions for the treatment and/or prevention of androgenetic alopecia (AGA) and /or hair loss.

EP 4 082 518 A1

## Description

### FIELD OF THE INVENTION

The present invention provides a fatty acid mixture with improved efficacy and inhibited gene expression of SRD5A1, SRD5A2 and SRD5A3 as well as compositions thereof suitable for the treatment and/or prevention of hair loss and alopecia, notably with lower dosage and low toxicity. Furthermore, the present invention also relates to the process to obtain said mixtures and composition and to a method for the treatment and/or prevention of androgenetic alopecia (AGA), which employs said compositions.

### BACKGROUND OF THE INVENTION

Androgenetic alopecia (AGA) or also known as Male-pattern hair loss (MPHL) is the most common type of hair loss, affecting women 50 % of menopausal women, around 25 % of women of childbearing age, as well as over 70 % of adult men, [McElwee and Saphiro, 2012]. It occurs due to an underlying susceptibility of hair follicles to shrinkage due to the combined effect of two factors: Genetic predisposition (several loci are involved including androgenic receptor (AR), EDA2R/Chr. X-WNT10A/2q35, etc.) and hormonal stimulation [Liang et al., 2013; Rinaldi et al., 2016]. It is known that both genetic and environmental factors play a role, but many causes of AGA remain unknown.

Androgens control the proliferation of human hair, which responds to hormones differently, depending on the body location [Thornton et al., 1991]. Dermal papilla cells (DPCs) of the beard, armpit and hair scalp of people, who are genetically predisposed to baldness, were shown to be androgen target cells [Randall, 2007].

It is known that the enzymes $5\alpha$-reductases convert testosterone to $5\alpha$-dihydrotestosterone (DHT) and this conversion enhances the androgenic signal via two mechanisms. In the first one, DHT cannot be aromatized to estrogen and therefore, its effect is solely androgenic. In the second one, in vitro DHT binds to the AR with a higher affinity than testosterone, thus preventing its normal action. Specifically, isoenzymes type 1 and 2 are highly present at pilosebaceuous units in papillae of individual hair follicles [Bernard, 1994]. For these reasons, $5\alpha$-reductase inhibitors (5-ARIs) have been used in the treatment of androgenic alopecia and can be beneficial in the treatments of the anti-hair loss due to androgenetic alopecia.

Serenoa repens extracts are known to be effective in the treatment of alopecia and to inhibit both types of 5-$\alpha$ reductase.

The role of fatty acid mixtures in a cosmetic composition for the treatment and prevention of hair loss and to reduce the impact of testosterone was investigated in FR2841101. The oral compositions of FR2841101 described cosmetic compositions comprising taurine, hypotaurine and mixtures of n-6 and n-3 polyunsaturated essential fatty acids of between 18-22 carbon atoms.

Later, EP16237408A1 described pharmaceutical composition obtained from the green or mature fruits of Roystonea regia, which contains a mixture of the fatty acids, including, caprilic (C8:0), capric (C10: 0), lauric (C12:0), miristic (C14:0), palmitic (C16:0), palmitoleic (C16:1), estearic (C18:0) and oleic (C18:1) and mixtures with esters thereof. The effect of said compositions and extracts were tested in the treatment of prostate hyperplasia PH and hair growth on mice and rats. However, said fatty acid compositions are obtained by extraction of the fatty acids form the plant by means of organic solvent extraction technology, providing very low yields of said extracts.

Subsequently, EP 2964326 B1 reported fat cream compositions comprising fatty acids derived from the hump of a camel to stimulate hair growth, said fat hump camel comprising fatty acids such as palmitic acid, stearic acid, oleic acid, mystiric acid or omega 3, 6 o 9. However, EP 2964326 B1 is silent regarding the amount of the fatty acids used and had the further disadvantage that the hump camel fat is not readily available.

Despite some progress being made in the recent years, there is an on-going need for composition suitable for the treatment or/and prevention of hair loss and alopecia with improved efficacy and inhibited gene expression of SRD5A1, SRD5A2 and SRD5A3, notably with lower dosage and low toxicity. Also, there is a need to develop an industrially feasible, more economical and environmentally friendly process for the preparation of pharmaceutical compositions suitable to treat or prevent alopecia and/or hair loss.

### BRIEF DESCRIPTION OF THE INVENTION

The first aspect of the invention is related to a fatty acid mixture composition characterised in that the composition comprises at least:

- Caprylic acid (C8:0) in a concentration from 2 up to 5% by weight;
- Capric acid (C10:0) in a concentration from 2 up to 5 % by weight;
- Lauric acid (C12:0) in a concentration from 30 up to 50 % by weight;

- Myristic acid (C14:0) in a concentration from 10 up to 20% by weight;
- Palmitic acid (C16:0) in a concentration from 4 up to 10 % by weight;
- Oleic acid (C18:1) in a concentration from 20 up to 33% by weight; and
- Linolenic acid (C18:3) in a concentration from 1 up to 10 % by weight.

The fatty acids mixture compositions of the first aspect can be isolated from natural sources such as, olive oil, sunflower oil, soy oil, palm oil, palm kernel oil, coconut oil, safflower oil, canola oil or mixtures thereof; said natural sources comprise: Caprilic acid (C8:0), Capric acid (C10:0), Láurico acid (C12:0), Mirístic acid (C14:0), Palmitic acid (C16:0), Oleico acid (C18:1), Linolenic acid (C18:3), or a derivative or combination thereof. The fatty acids can be isolated using standard methods know in the art or van be used directly from said natural source by using the adequate amount. The fatty acids of the first aspect can be also purchase individually as they are commercially abalialbe for cosmetic and pharmaceutical grade.

The fatty acids mixture compositions of the first aspect have the advantage that show high inhibition of the gene expression of all 5$\alpha$-Reductase, SRD5A1 (5$\alpha$-Reductase type 1), SRD5A2 (5$\alpha$-Reductase type 2) and SRD5A3 (5$\alpha$-Reductase type 3), showing also an improvement versus the known fatty acid compositions of the prior art.

SRD5A1 (3-oxo-5$\alpha$-steroid 4-dehydrogenase 1) is an enzyme that in humans is encoded by the SRD5A1 gene.

SRD5A2 (3-oxo-5a-steroid 4-dehydrogenase 2) is an enzyme that in humans is encoded by the SRD5A2 gene.

SRD5A3, (Steroid 5-alpha-reductase 3 or Polyprenol reductase), also known as 3-oxo-5-alpha-steroid 4-dehydrogenase 3, is an enzyme that in humans is encoded by the SRD5A3 gene.

Likewise, the fatty acids mixture compositions of the first aspect show very good toxicity profile allowing the use of high concentrations of said mixtures in pharmaceutical and cosmetic compositions, showing a very good toxicity profile when compared to the known compositions of the prior art. Equally, the fatty acids mixture compositions of the first aspect, showed a very good efficacy when tested at low concentrations as well as higher concentrations, which allows the reduction of the % of the fatty acid mixture in the pharmaceutical or cosmetic product ensuring safety and efficacy.

The fatty acids mixture compositions of the first aspect, have been proven to be suitable and readily easy to be used in pharmaceutical or cosmetic compositions for the treatment or prevention of alopecia or heir loss.

In a second aspect, the invention relates to a pharmaceutical or cosmetic composition, which comprises the fatty acid mixture composition according to the first aspect and at least one pharmaceutical or cosmetic acceptable excipient and optionally at least an active ingredient.

The pharmaceutical or cosmetic compositions of the second aspect are effective when tested at very low dosage, showing high inhibition of the gene expression of all 5$\alpha$-Reductase, SRD5A1, SRD5A2 and SRD5A3 and no toxicity.

In a third aspect, the invention refers to a method to prepare the composition according to the first aspect, which comprises at least the steps of:

a) Mixing the fatty acids according to the fatty acid composition of the first aspect to provide a fatty acid mixture;
b) mixing the fatty acid mixture obtained in the previous step with water and at least one surfactant followed by stirring at a speed range from 200 rpm to 10.000 rpm to provide a water in oil emulsion;
c) drying the emulsion obtained in the previous step, preferably by spray drying, to provide the composition of the first aspect, preferably in the form of a powder.

In a fourth aspect, the invention relates to the use of the fatty acid mix composition according to the first aspect for the preparation of a cosmetic composition, wherein the cosmetic composition is suitable to treat or prevent alopecia.

In a fifth aspect, the invention relates to a pharmaceutical composition according the second aspect, for use in a method for the treatment and/or prevention of androgenetic alopecia (AGA), reduction of hair lost, pattern baldness, benign prostatic hyperplasia or hirsutism, preferably wherein the method comprises oral, topical or subcutaneous administration of said composition to a human subject.

## DETAILED DESCRIPTION OF THE INVENTION

The first aspect of the invention is related to a fatty acid mixture composition characterised in that the composition comprises at least:

- Caprylic acid (C8:0) in a concentration from 2 up to 5% by weight;
- Capric acid (C10:0) in a concentration from 2 up to 5 % by weight;
- Lauric acid (C12:0) in a concentration from 30 up to 50 % by weight;
- Myristic acid (C14:0) in a concentration from 10 up to 20% by weight;

- Palmitic acid (C16:0) in a concentration from 4 up to 10 % by weight;
- Oleic acid (C18:1) in a concentration from 20 up to 33% by weight; and
- Linolenic acid (C18:3) in a concentration from 1 up to 10 % by weight.

In a preferred embodiment of the fatty acid mixture composition of the first aspect, the fatty acid mixture composition comprises at least:

- Caprylic acid (C8:0) in a concentration from 2 up to 5 % by weight
- Capric acid (C10:0) in a concentration from 2 up to 5 % by weight
- Lauric acid (C12:0) in a concentration from 30 up to 45 % by weight
- Myristic acid (C14:0) in a concentration from 10 up to 18% by weight
- Palmitic acid (C16:0) in a concentration from 5 up to 9 % by weight
- Oleic acid (C18:1) in a concentration from 23 up to 33% by weight
- Linolenic acid (C18:3) in a concentration from 2-7%

For the sake of clarity, the reference (Cn:Y) used identify the different fatty acids, relates to the number of carbon atoms, wherein n is the number of C atoms in the fatty acid and Y is the number of double bounds present in the fatty acid.

In a preferred embodiment of the composition of the first aspect, the fatty acid mixture comprises Caprylic acid (C8:0) in a concentration from 2 up to 5 % by weight and/or Capric acid (C10:0) in a concentration from 2 up to 5 % by weight. These preferred ranges show good efficacy and better properties, such as a better inhibition of the expression of SRD5A1, SRD5A2 and/or SRD5A3.

In another preferred embodiment of the composition of the first aspect, the fatty acid mixture comprises Linolenic acid (C18:3) in a concentration from 2-7%. These preferred ranges show good efficacy and better properties, such as a better inhibition of the expression of SRD5A1, SRD5A2 and/or SRD5A3.

In another preferred embodiment of the composition of the first aspect, the fatty acid mixture composition further comprises stearic acid (C18:0) in a concentration from 2 up to 4% by weight.

In another preferred embodiment of the composition of the second aspect, the fatty acid mixture composition does not comprise palmitoleic and/or linoleic acid, said fatty acid mixtures show good efficacy and better properties, such as a better inhibition of the expression of SRD5A1, SRD5A2 and SRD5A3.

The fatty acid compositions of the first aspect, can be an improve treatment or/and an alternative treatment for male pattern baldness in male patients who do not want or cannot tolerate the side-effects of standard medications.

The fatty acid compositions of the first aspect, show the advantage that can be formulated as pharmaceutical and cosmetic compositions containing low concentrations of the fatty acid mix of the first aspect as well as showing high efficacy. Likewise, as the daily dosages can be low, the toxicity effects are eliminated or significantly reduced. Furthermore, as the affective dosage is low, this allows the manufacturing of oral compositions with low dimensions, improving the swallowing of said compositions by the subjects and a better adhesion to the treatments or regimes by any subject in need.

In a second aspect, the invention relates to a pharmaceutical or cosmetic composition, which comprises the fatty acid mixture composition according to the first aspect and at least one pharmaceutical or cosmetic acceptable excipient and optionally at least an active ingredient.

In a preferred embodiment of the composition of the second aspect, the fatty acid mixture is present in the composition of the second aspect in a concentration from 10 up to 40%, preferably from 15 to 30%. The compositions of the second aspect, have the advantage that they have high efficacy, low toxicity and/or lack of side effects.

The term "excipient" or "pharmaceutically acceptable excipient" refers to carriers, excipients, or vehicles such as those described herein and in Remington's Pharmaceutical Sciences 22th edition (2013).

Suitable pharmaceutical or cosmetic acceptable excipients can be selected from; diluents, preservative, disintegrants, lubricants, binders and glidants.

Suitable "diluents" or "fillers" may be selected form the list consisting of, water, ethanol, isopropanol, propylene glycol, acetone, cellulose, hydroxypropyl methylcellulose, glyceryl monostearate, modified starch, Carbopol, sorbitol, sucralose, xylitol, gelatin, glycerin/glycerol, triethanolamine, mineral oils and natural oils.

Suitable "lubricants" may be selected form the list consisting of, magnesium stearate, stearic acid, silicones, dimethicones, cycle-silicones and lanolin, acetylated lanolin alcohol.

Suitable "preservatives or stabilisers" may be selected form the list consisting of, dicalcium phosphate, calcium carbonate, disodium phosphate, potassium sorbate, tricalcium phosphate, modified starch, copper chlorophyllin, sulfur dioxide, sorbitol and iron oxide.

Suitable " disintegrants" may be selected form the list consisting of, crosslinked cellulose gum (croscarmellose) and crospovidone.

Suitable "Glidants" may be selected from form the list consisting of, colloidal silica such as Aerosil 200 or Aerosil R972, silicates, titanium dioxide and silicon dioxide.

Suitable "binders" may be selected from form the list consisting of, Acacia, Carragenano, polyvinyl pyrrolidone (PVP).

In another preferred embodiment of the second aspect, the pharmaceutical or cosmetic composition is an oral composition, preferably in the form of tablets, soft capsules, hard capsules or as a powder.

In preferred embodiment of the second aspect, the pharmaceutical or cosmetic composition comprises the fatty acid mixture of the first aspect in an amount from 50 mg to 500 mg or in an amount from 100 to 400 mg. More preferably in an amount from 100 to 200 mg.

In another preferred embodiment of the second aspect, the pharmaceutical or cosmetic composition, is an oral composition in the form of a hard capsules. Preferably, the pharmaceutical or cosmetic composition of the second aspect is administered at a daily dose of the fatty acid mix of the first aspect from 50 to 1000 mg/day. In another preferred embodiment the daily dose of the fatty acid mix of the first aspect is from 100 to 600 mg/day or from 150 to 350 mg/day.

In a third aspect, the invention refers to a method to prepare the composition according to the first aspect, which comprises at least the steps of:

a) Mixing the fatty acids according to the fatty acid composition of the first aspect to provide a fatty acid mixture;
b) mixing the fatty acid mixture obtained in the previous step with water and at least one surfactant followed by stirring at a speed range from 200 rpm to 10.000 rpm to provide a water in oil emulsion;
c) drying the emulsion obtained in the previous step, preferably by spray drying, to provide a composition in the form of a powder.

The fatty acids of step a) can be obtained from an oil source or can be individually purchase to provide the mixture of step a) in the required concentrations according to the first aspect.

In a preferred embodiment of the third aspect, the fatty acids can be obtained from an oil source, wherein the oils source is selected from the list consisting of, olive oil, sunflower oil, soy oil, palm oil, palm kernel oil, coconut oil, safflower oil and canola oil. The amount of the different oils source is adjusted and calculated to provide the necessary amount of each of the fatty acids as described in the first aspect, by means of the standard methods of the common general knowledge.

In another preferred embodiment of the third aspect the drying step c) is carried out by means of spray drying using a hot gas to provide a fast drying and a well dried and stable powder. The step c) improves the stability of the fatty acid dry powder mixture obtained, particularly versus oxidation.

In another preferred embodiment of the third aspect, the surfactant can be selected from polyoxyethyl sorbitan esters (sorbitol and its copolymerized anhydrides with 4, 5, or 20 moles of ethylene oxide) partially esterified with superior fatty acids and from sorbitan monolaureate, sorbitan esters (also referred as spans) or mixtures thereof. The term tween, polysorbate or polyoxyethyl sorbitan esters can be used indistinguishably. Suitable polyoxyethyl sorbitan esters or polysorbates are selected form the list consisting of; polysorbate 20 (Polyoxyethylethane monolaurate-(4)-sorbitan), 21 (Polyoxyethyl monolaurate-(8)-sorbitane), 22 (Polyoxyethane monolaurate-(12)-sorbitan), 23 (Polyoxyethylen monolaurate-(16)-sorbitan).

Suitable Spans or sorbitan esters are selected from, Sorbitan Triestearate (Span 60), Sorbitan Monolaurate (Span 20), Sorbitan Monooleate (Span 80), Sorbitan Monopalmitate (Span 40) and Sorbitan Trioleate (Span 85).

In another preferred embodiment of the third aspect, the amount of surfactant used in of step b) is sufficient to reach a Critical Micell Concentration (CMC) from 0.3 to 1mM, preferably from 0.35 to 0.8 mM. The term Critical Micell Concentration (CMC) is defined as the concentration of surfactants above which micelles form and all additional surfactants added to the system will form micelles. The concentration of the fatty acid mixture of step a) in water may be from 10% up to 50%.

In a fourth aspect, the invention relates to the use of the fatty acid mix composition according to the first aspect for the preparation of a cosmetic composition, wherein the cosmetic composition is suitable to treat or prevent alopecia.

In a fifth aspect, the invention relates to a pharmaceutical composition according the second aspect, for use in a method for the treatment and/or prevention of androgenetic alopecia (AGA), reduction of hair lost, pattern baldness, benign prostatic hyperplasia or hirsutism, preferably wherein the method comprises oral, topical or subcutaneous administration of said composition on a human subject.

## EXAMPLES

The following examples serve to illustrate the present invention and should not be construed as limiting the scope thereof.

For these reasons, in this assay, 2 different composition of the invention were tested and compared to an extract of Serenoa repens (comparative example 1) and to an extract of d extract of Roystonea regia according to table 2 of example 1 and table 5 of example 4 of EP 1 623 740 81A (referred to comparative example 2, 3) to inhibit the expression of SRD5A1 ($5\alpha$-Reductase type 1), SRD5A2 ($5\alpha$-Reductase type 2) and SRD5A3 ($5\alpha$-Reductase type 3) through RT-qPCR. The effect was assessed in vitro after 24 hours of treatment of the above mixtures in the Human Follicle Dermal Papilla Cells (HFDPC). All samples were stored at room temperature to avoid alteration until the start of the experiment and dilutions were freshly prepared each time.

METHODOLOGY: For gene expression analysis, HFDPC cells were treated with the mixtures described in table 1 for 24 hours using 2 different concentrations, 0.001 % and 0.005 %. Afterwards, the total RNA was purified, quantified and it was used to synthesize complementary DNA (cDNA). This obtained cDNA from treated or untreated cells (control cells) was used to determine the relative gene expression of SRD5A1, SRD5A2 and SRD5A3 through RT-qPCR. Actin (ACT) was used as reference gene. The obtained data was statistically analysed.

The tested composition as are described in table 1

Table 1: qualitative and quantitative compositions tested (in weight %).

| Fatty acids | Weight % in the fatty acid mixture | | | | |
|---|---|---|---|---|---|
| | comparative example 1 Serenoa repens | Comparative example 2 Roystonea regia (table 2 of example 1 of EP162374081A) | Comparative example 3 Roystonea regia (table 5 of example 4 of EP162374081A) | Example 1 | Example 2 |
| Caprylic acid | 2.3 | 1 | 1 | 3.2 | 4 |
| Capric acid | 2.6 | 1 | 1 | 2.8 | 3.4 |
| Lauric acid | 29 | 5.7 | 19.7 | 34.7 | 36.5 |
| Myristic acid | 11.1 | 4.4 | 9.7 | 12.5 | 13 |
| Palmitoleic acid | 0.6 | 1.9 | 0.2 | -- | -- |
| Palmitic acid | 8.8 | 75.1 | 14.8 | 7.5 | 5 |
| Linoleic polyunsaturated acid | 4.6 | -- | -- | -- | -- |
| Linolenic acid | 1.1 | -- | -- | 4.5 | 5 |
| Oleic acid | 33 | 9.10 | 49.8 | 31.3 | 31 |
| Stearic acid | 1.9 | 1.9 | 3.8 | 2.6 | 2.1 |
| Sterols | 0.4 | -- | -- | -- | -- |
| $\beta$-Sitosterol | 0.3 | -- | -- | -- | -- |
| Fatty Alcohols | 0.3 | -- | -- | -- | -- |

**Preparation of compositions of example 1 and 2.**

The compositions of example 1 and 2 were prepared by mixing a fatty acid of examples 1 or 2 in the appropriate amount. Water and Tween 20 is added to the fatty acid mixture (30% in water) obtained in the previous step and stirred at a speed of 3000 rpm for 1 h at a temperature of 40 °C. Afterwards, the emulsion obtained in step b) was dried by means of spray drying using a hot gas to provide a fast drying and a well dried and stable powder.

The composition of the second aspect was prepared by mixing the powder of examples 1 or 2 obtained in the previous example with the excipients described in the second aspect to provide a hard capsule comprising 150 mg of fatty acid mixture.

**Analytical equipment.**

Stereoscopic microscope, incubator, refrigerated centrifuge, statistical analysis software, laminar flow hood, Bürker chamber, micropipettes, tips, pipettes, propipette, rack, quantifier Nano-Drop spectrophotometer, Quant studio 5 (Applied Biosystem) Quantitative real-time PCR, vortex, precision balance, heating block and consumables.

Reagents HFDPC culture medium (Promocell), nutrient solution mix (Promocell), Phosphate buffered saline (Sigma), Trypan Blue Solution (Bio-Rad), Ethanol (Sigma-Aldrich), MTT reagent (Invitrogen), DMSO (SIGMA), RNAse free-DNAse (Qiagen), PrimeScript RT Reagent (Perfect Real Time- Takara Clontech), Oligonucleotides for RT-PCR amplification of SRD5A1, SRD5A2, SRD5A3 and β-ACT, SYBR ® qRT-PCR, liquid nitrogen.

**Procedure**

For seeding cells, cell numbers and viability were determined using Trypan-Blue staining and counting in a Bürker chamber under the microscope. For the main gene expression assay, HFDPC cells were cultured at a 300.000 cells/well density in a 6-well plate, in growth medium. 24 hours later, the medium was removed and different mixtures of table 1, prepared at 0.001 % and 0.005 % concentration were added to cells. After 24 hours of incubation period, cells were washed with PBS buffer and collected in lysis buffer to proceed with RNA extraction. Total RNA was extracted using RNeasy kit (Qiagen) and treated with DNAse-I to remove any contamination from genomic DNA. RNA quality and quantity were checked in a Nano-Drop spectrophotometer, and 500 μg of total RNA was used to synthesize cDNA, using First-strand Synthesis kit (Takara-Clontech). The suitability of each primer pair used in this study for RT-qPCR, SRD5A1, SRD5A2, SRD5A3 and ACT was previously evaluated to determine melting curves, efficiency of amplification and specificity of the primers. Finally, quantitative PCR (qPCR) was performed in a real time PCR machine (QuantStudio 5, Applied BioSystem). To perform raw data analysis, the Pfaffl method [Pfaffl, 2001] was used to calculate the gene relative expression ratio to ACT (internal control- housekeeping gene). Mathematical model of relative expression ratio in real-time PCR was used. Statistical analysis to determine significant changes was performed using Student's t-test. For all data a level of 5% or less ($p < 0.05$) was taken as statistically significant.

Mathematical model of relative expression ratio used in real-time PCR data analysis. The ratio of a target gene is expressed in a sample versus a control in comparison to a reference gene.

$$ratio = \frac{(E_{target})^{\Delta CP\ target\ (control-sample)}}{(E_{ref})^{\Delta CP\ ref\ (control-sample)}}$$

Etarget is the real-time PCR efficiency of target gene transcript; Eref is the real-time PCR efficiency of a reference gene transcript; ΔCPtarget is the CP deviation control - sample of the target gene transcript; ΔCPref = CP deviation of control - sample of reference gene transcript.

**Gene expression quantification by qPCR**

mRNA expression levels were determined after treatment with the product of table 1 tested in Human Follicle Dermal Papilla Cells (HFDPC) for 24 hours. SRD5A1, SRD5A2, SRD5A3 and ACT (internal control) were amplified using four technical replicates of cDNAs.

The results showing SRD5A1, SRD5A2 and SRD5A3 gene expression values after treating HFDPC cells with the mixtures of table 1 are listed in table 2

Table 2: test results regarding inhibition of gene expression of the tested formulations.

| *SRD5A 1 - RT-qPCR* | | |
|---|---|---|
| **Dunnett's multiple comparisons test** | **Mean Difference** | **95% Confidence interval** |
| Control vs. Example 1 (0.001%) | -0,1095 ± 0,07159 | -0,1965 to 0,4155 |
| Control vs. Serenoa (0.001 %) Comparative example 1 | -0,02425 ± 0,1147 | -0,2983 to 0,3468 |
| Control vs. Roystonea Regia (0.001%) Comparative example 2 | 0,3414 ± 0,1123 | -0,6513 to -0,03161 |
| Control vs. Roystonea Regia (0.001%) Comparative example 3 | 0,5318 ± 0,1010 | -0,8664 to -0,1971 |

(continued)

| SRD5A2-RT-qPCR | | |
|---|---|---|
| Dunnett's multiple comparisons test | Mean Difference | 95% Confidence interval |
| Control vs. Example 1 (0.001 %) | -0,4380 ± 0,1376 | 0,03406 to 0,8419 |
| Control vs. Serenoa (0.001 %) Comparative example 1 | -0,3376 ± 0,1665 | -0,1049 to 0,7801 |
| Control vs. Roystonea Regia (0.001 %) Comparative example 2 | 1,508 ± 0,2233 | 1,052 to 1,964 |
| Control vs. Roystonea Regia (0.001%) Comparative example 3 | -0,04606 ± 0,09617 | -0,2437 to 0,1516 |
| Control vs. Example 1 (0.005%) | -0,09400 ± 0,2353 | -0,6365 to 0,4485 |
| Control vs. Roystonea Regia (0.005%) Comparative example 2 | 0,4823 ± 0,1498 | 0,1764 to 0,7882 |
| Control vs. Roystonea Regia (0.005%) Comparative example 3 | 1,604 ± 0,1589 | 1,279 to 1,929 |
| SRD5A3 - RT-qPCR | | |
| Dunnett's multiple comparisons test | Mean Difference | 95% Confidence interval |
| Control vs. Example 1 (0.001%) | -0,3985 ± 0,1451 | 0,06844 to 0,7286 |
| Control vs. Serenoa (0.001 %) Comparative example 1 | -0,3498 ± 0,1639 | 0,01978 to 0,6799 |
| Control vs. Roystonea Regia (0.001%) Comparative example 2 | 0,09050 ± 0,1359 | -0,1871 to 0,3681 |
| Control vs. Roystonea Regia (0.001%) Comparative example 3 | 1,156 ± 0,5764 | -0,03956 to 2,351 |
| Control vs. Example 1 (0.005%) | -0,6015 ± 0,1017 | 0,2884 to 0,9146 |
| Control vs. Serenoa (0.005%) Comparative example 1 | -0,5778 ± 0,1138 | 0,2646 to 0,8909 |
| Control vs. Roystonea Regia (0.005%) Comparative example 2 | 1,966 ± 0,3327 | 1,286 to 2,645 |
| Control vs. Roystonea Regia (0.005%) Comparative example 3 | -0,06569 ± 0,1584 | -0,3892 to 0,2578 |

Test results indicated that treatment with example 1 of the invention at 0.001 % concentration, significantly inhibited gene expression of SRD5A2 and SRD5A3 by 43.8 ± 13.8 % and 39.9 ± 14.5 %, respectively, compared to the untreated control, whereas the treatment at 0.005 % significantly decreased gene expression of SRD5A3 by 60.2 ± 10.2 %. Likewise, the treatment with example 1 of the invention both at 0.001 % and 0.005% concentration showed a greater inhibition of SRD5A1, SRD5A2 and SRD5A3 for all Roystonea regia samples, comparative example 2 ad comparative example 3. The inhibited gene expression for all SRD5A1, SRD5A2 and SRD5A3 of the example 1 of the invention, were particularly good for all concentrations tested and even better for the lower concentration, 0001%, of the fatty acid mixture. This reduces or eliminates any toxicity of the pharmaceutical or cosmetic compositions thereof and treatments thereof.

Roystonea regia, comparative example 2 and comparative example 3 showed no improvement regarding inhibition of SRD5A1, SRD5A2 or SRD5A3 at both concentrations 0.001% or 0.005%.

**Cell viability (MTT)**

The cell viability of the 24 hours treatment of Example 1 of the invention indicated that safe working concentrations would start at 0.01 % or even 0.1% or 0.3% and the slight toxicity detected at these concentrations and lower concentrations is considered irrelevant. Distinctively, it should be noted that the cell viability for the Serenoa repens (comparative example 1) at product concentrations above 0.01% showed very poor OD600 readings and bad cell viability.

**Claims**

1. A fatty acid mixture composition **characterised in that** the composition comprises at least:

   - Caprylic acid (C8:0) in a concentration from 2 up to 5% by weight;
   - Capric acid (C10:0) in a concentration from 2 up to 5 % by weight;
   - Lauric acid (C12:0) in a concentration from 30 up to 50 % by weight;
   - Myristic acid (C14:0) in a concentration from 10 up to 20% by weight;
   - Palmitic acid (C16:0) in a concentration from 4 up to 10 % by weight;
   - Oleic acid (C18:1) in a concentration from 20 up to 33% by weight; and
   - Linolenic acid (C18:3) in a concentration from 1 up to 10% by weight.

2. The composition according to the previous claim, wherein

   - Caprylic acid (C8:0) in a concentration from 2 up to 5 % by weight
   - Capric acid (C10:0) in a concentration from 2 up to 5 % by weight
   - Lauric acid (C12:0) in a concentration from 30 up to 45 % by weight
   - Myristic acid (C14:0) in a concentration from 10 up to 18% by weight
   - Palmitic acid (C16:0) in a concentration from 5 up to 9 % by weight
   - Oleic acid (C18:1) in a concentration from 23 up to 33% by weight
   - Linolenic acid (C18:3) in a concentration from 2-7%

3. The composition according to any of the preceding claims, wherein the composition further comprises stearic acid (C18:0) in a concentration from 2 up to 4% by weight.

4. The composition according to the any of the preceding claims, wherein the composition does not comprise palmitoleic and/or linoleic acid.

5. A pharmaceutical or cosmetic composition, which comprises the fatty acid mixture composition according to claims 1-4 and at least one pharmaceutical or cosmetic acceptable excipient and optionally at least an active ingredient.

6. The pharmaceutical or cosmetic composition according to the previous claim, wherein the fatty acid mixture composition according to claims 1-4 is present in a weight percentage from 10-40% of the total weight of the composition.

7. The pharmaceutical or cosmetic composition according to any of the claims 5-6, wherein the composition is in the form of a liquid, a gel, a lotion, or a cream composition, a solid composition, wherein the liquid composition is selected from the list consisting of, a solution, a dispersion, an emulsion and a suspension.

8. The pharmaceutical or cosmetic composition according to claim 7, wherein the solid composition is selected from the list consisting of, tablets, hard capsules, soft capsules, granules or powder wherein the composition is administered at a daily dose from 50 mg to 1000 mg, preferably from 100 to 600 mg, of the fatty acid composition according to claims 1-4.

9. The pharmaceutical or cosmetic composition according to any of the claims 5-7, wherein the composition comprises from 50 mg to 500 mg, preferably from 100 to 400 mg, of the fatty acid mixture composition according to claims 1-4 and the composition is administered from once a day up to 3 times a day.

10. The composition according to any of the claims 1-4 or the pharmaceutical composition according to any of the claims 5-9, for use in a method to increase expression of SRD5A1, SRD5A2 and/or SRD5A3.

11. The pharmaceutical composition according to any of the claims 5-10 for use in a method to treat and/or prevent alopecia and/or hair loss.

12. A method to prepare the composition according to any of the claims 5-11 which comprises at least the steps of:

a) Mixing the fatty acids according to claims 1-4 to provide a fatty acid mixture;

b) mixing the fatty acid mixture obtained in the previous step with water and at least one surfactant followed by stirring at a speed range from 200 rpm to 10.000 rpm to provide a water in oil emulsion;

c) drying the emulsion obtained in the previous step, preferably by spray drying, to provide a composition in the form of a powder.

13. The method according to the previous claim, wherein the fatty acids of step a) are obtained from an oil source, wherein the oils source is selected from the list consisting of, olive oil, sunflower oil, soy oil, palm oil, palm kernel oil, coconut oil, safflower oil and canola oil.

14. The use of the fatty acid mix composition according to any of the claims 1-4 for the preparation of a cosmetic composition, wherein the cosmetic composition is suitable to treat or prevent alopecia.

15. The pharmaceutical composition according to any of the claims 5-9, for use in a method for the treatment and/or prevention of androgenetic alopecia (AGA), reduction of hair lost, pattern baldness, benign prostatic hyperplasia or hirsutism, preferably wherein the method comprises oral, topical or subcutaneous administration of said composition on a human subject.

Europäisches Patentamt

European Patent Office

Office européen des brevets

**EUROPEAN SEARCH REPORT**

Application Number

EP 21 38 2370

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ES 2 517 741 A1 (LACER SA [ES]) 3 November 2014 (2014-11-03) * example 1 * * paragraph [0001] - paragraph [0015] * * paragraph [0023] - paragraph [0049] * * paragraph [0065] - paragraph [0066] * * paragraph [0096] * * paragraph [0115] * ----- | 1-15 | INV. A61K8/36 A61Q7/00 |
| X | KWON YOUNGJOO: "Use of saw palmetto () extract for benign prostatic hyperplasia", FOOD SCIENCE AND BIOTECHNOLOGY, THE KOREA SOC. OF FOOD SCIENCE AND TECHNOLOGY, HEIDELBERG, vol. 28, no. 6, 17 April 2019 (2019-04-17), pages 1599-1606, XP036945288, ISSN: 1226-7708, DOI: 10.1007/S10068-019-00605-9 [retrieved on 2019-04-17] | 1,2,4,5, 11,14,15 | |
| Y | * page 1601 - page 1604; table 1 * ----- | 1-15 | |
| Y | US 2015/190358 A1 (CANAVACIOLO VICTOR LUIS GONZALES [CU] ET AL) 9 July 2015 (2015-07-09) * claims 1-23; tables 1-6 * ----- | 1-15 | TECHNICAL FIELDS SEARCHED (IPC) A61K A61Q |
| Y | US 2019/275122 A1 (SHLIEOUT GEORGE [DE] ET AL) 12 September 2019 (2019-09-12) * paragraph [0120] - paragraph [0124] * ----- | 1-15 | |
| Y | US 2013/122128 A1 (KIM BONGCHEOL [KR] ET AL) 16 May 2013 (2013-05-16) * paragraph [0094] * * paragraph [0100] - paragraph [0105] * ----- | 1-15 | |
| Y | WO 94/01100 A1 (ARCH DEV CORP [US]) 20 January 1994 (1994-01-20) * the whole document * ----- | 1-15 | |

The present search report has been drawn up for all claims

| Place of search | Date of completion of the search | Examiner |
|---|---|---|
| Munich | 16 November 2021 | Steinheimer, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 38 2370

This annex lists the patent family members relating to the patent documents cited in the above-mentioned European search report.
The members are as contained in the European Patent Office EDP file on
The European Patent Office is in no way liable for these particulars which are merely given for the purpose of information.

16-11-2021

| Patent document cited in search report | | Publication date | Patent family member(s) | | Publication date |
|---|---|---|---|---|---|
| ES 2517741 | A1 | 03-11-2014 | NONE | | |
| US 2015190358 | A1 | 09-07-2015 | AU | 2013279850 A1 | 29-01-2015 |
| | | | BR | 112014031826 A2 | 27-06-2017 |
| | | | CA | 2876907 A1 | 27-12-2013 |
| | | | CN | 104684544 A | 03-06-2015 |
| | | | CO | 7240409 A2 | 17-04-2015 |
| | | | CU | 20120097 A7 | 29-01-2014 |
| | | | DK | 2862567 T3 | 06-11-2017 |
| | | | EP | 2862567 A2 | 22-04-2015 |
| | | | ES | 2648489 T3 | 03-01-2018 |
| | | | JP | 6134953 B2 | 31-05-2017 |
| | | | JP | 2015521606 A | 30-07-2015 |
| | | | KR | 20150029710 A | 18-03-2015 |
| | | | MX | 361904 B | 19-12-2018 |
| | | | PH | 12014502817 A1 | 23-02-2015 |
| | | | PT | 2862567 T | 27-11-2017 |
| | | | US | 2015190358 A1 | 09-07-2015 |
| | | | WO | 2013189467 A2 | 27-12-2013 |
| US 2019275122 | A1 | 12-09-2019 | AT | 407693 T | 15-09-2008 |
| | | | AU | 2005227090 A1 | 06-10-2005 |
| | | | BR | PI0509148 A | 11-09-2007 |
| | | | CA | 2560613 A1 | 06-10-2005 |
| | | | CN | 1933850 A | 21-03-2007 |
| | | | EP | 1729797 A1 | 13-12-2006 |
| | | | ES | 2314646 T3 | 16-03-2009 |
| | | | HK | 1101342 A1 | 18-10-2007 |
| | | | JP | 5032300 B2 | 26-09-2012 |
| | | | JP | 2007530503 A | 01-11-2007 |
| | | | PL | 1729797 T3 | 27-02-2009 |
| | | | PT | 1729797 E | 17-12-2008 |
| | | | RU | 2381813 C2 | 20-02-2010 |
| | | | US | 2005250817 A1 | 10-11-2005 |
| | | | US | 2014302000 A1 | 09-10-2014 |
| | | | US | 2019275122 A1 | 12-09-2019 |
| | | | WO | 2005092370 A1 | 06-10-2005 |
| US 2013122128 | A1 | 16-05-2013 | US | 2010330214 A1 | 30-12-2010 |
| | | | US | 2013122128 A1 | 16-05-2013 |
| WO 9401100 | A1 | 20-01-1994 | EP | 0652749 A1 | 17-05-1995 |
| | | | JP | H08501771 A | 27-02-1996 |
| | | | TW | 290457 B | 11-11-1996 |
| | | | US | 5422371 A | 06-06-1995 |
| | | | WO | 9401100 A1 | 20-01-1994 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 21 38 2370

This annex lists the patent family members relating to the patent documents cited in the above-mentioned European search report.
The members are as contained in the European Patent Office EDP file on
The European Patent Office is in no way liable for these particulars which are merely given for the purpose of information.

16-11-2021

| Patent document cited in search report | Publication date | Patent family member(s) | Publication date |
|---|---|---|---|
| -------------------------------------------------------------------------------- | | | |

EPO FORM P0459

For more details about this annex : see Official Journal of the European Patent Office, No. 12/82

page 2 of 2

**REFERENCES CITED IN THE DESCRIPTION**

**Patent documents cited in the description**

- FR 2841101
- EP 16237408 A1

- EP 2964326 B1
- EP 162374081 A

**Non-patent literature cited in the description**

- Remington's Pharmaceutical Sciences. 2013